(19) Europäisches Patentamt
European Patent Office
Office européen des brevets

(11) Publication number: **0 485 952 A2**

(12) **EUROPEAN PATENT APPLICATION**

(21) Application number: **91119272.2**

(22) Date of filing: **12.11.91**

(51) Int. Cl.[5]: **C07D 207/22**

(30) Priority: **13.11.90 JP 308093/90**

(43) Date of publication of application: **20.05.92 Bulletin 92/21**

(84) Designated Contracting States: **AT BE CH DE DK ES FR GB GR IT LI LU NL SE**

(71) Applicant: **SHIONOGI SEIYAKU KABUSHIKI KAISHA**
**1-8, Doshomachi 3-chome Chuo-ku**
**Osaka 541(JP)**

(72) Inventor: **Tsushima, Tadahiko**
**1-18-13, Fushiodai**
**Ikeda-shi, Osaka(JP)**
Inventor: **Okada, Tetsuo**
**4-6-6, Akasakadai**
**Sakai-shi, Osaka(JP)**
Inventor: **Nishitani, Yasuhiro**
**49-16, Aobadai**
**Izumi-shi, Osaka(JP)**

(74) Representative: **Vossius & Partner**
**Siebertstrasse 4 P.O. Box 86 07 67**
**W-8000 München 86(DE)**

(54) **Process for production of 3-amino-4-methylenepyrrolidine derivatives.**

(57) The present invention relates to processes for the production of exo-methylene compounds of the formula:

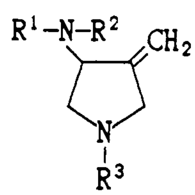

wherein $R^1$ and $R^3$ each is an amino-protecting group, and $R^2$ is hydrogen or a lower alkyl, which is characterized by methylenation of ketones of the formula:

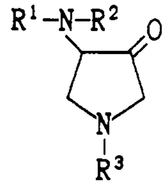

wherein $R^1$, $R^2$, and $R^3$ are the same as defined above.

The process is safe because no unusual and harmful organometallic reagent such as N-phenylselenophthalimide has to be used, and convenient for commercial mass production.

EP 0 485 952 A2

This invention relates to a process for the production of 3-amino-4-methylenepyrrolidine derivatives and, more particularly, to a process for the production of pyrrolidine derivatives which are to be introduced into pyridonecarboxylic acids, useful antibacterials, at the 7-position.

US-A-5,017,581 describes useful antibacterials, i.e., pyridonecarboxylic acids of the formula:

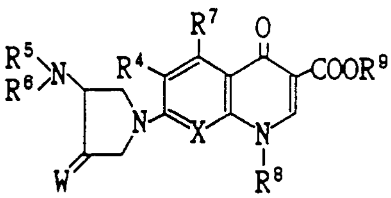

wherein $R^4$ is halogen; $R^5$ and $R^6$ each is identically or differently hydrogen or $C_1$-$C_3$ alkyl: $R^7$ is hydrogen, hydroxy, or amino; $R^8$ is $C_1$-$C_3$ alkyl or $C_3$-$C_7$ cycloalkyl; $R^9$ is hydrogen or a protecting group; W is $C_1$-$C_3$ alkylidene; X is N or C-Y; Y is hydrogen or halogen, and pharmaceutically acceptable salt thereof. They have high antibacterial activities with a wide spectrum. At that time, the 3-amino-4-methylenepyrrolidine moiety (W is $CH_2$) was prepared by a process using the following phenylseleno compound:

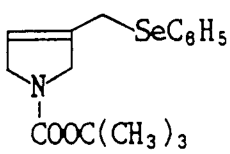

The conventional process for the production of the moiety at 7-position by way of the phenylseleno compound, which is disclosed in US-A-5,017,581 as stated before, requires unusual and harmful organometallic reagents such as N-phenylselenophthalimide. Therefore, the process is not satisfactory for commercial mass production in terms of simplicity and safety.

It is the object of the invention to provide a novel process for the production of exo-methylene compounds of the formula:

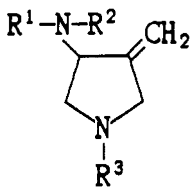

wherein $R^1$ and $R^3$ each is an amino-protecting group, and $R^2$ is hydrogen or a lower alkyl.

This object could be achieved on the basis of the finding that 3-amino-4-oxopyrrolidine derivatives (II) of the formula:

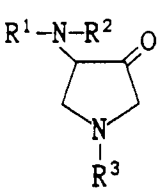
( II )

wherein $R^1$, $R^2$, and $R^3$ are as described above, can be the key-intermediate for the production of the exo-methylene compoumds. A methylene group can be easily introduced at 4-position, if the compounds (II) are employed as starting materials. The process using a compound (II) is safe because no unusual and harmful reagents are required, and it is convenient for commercial mass production.

Thus, the subject-matter of this invention in a novel process for the production of exo-methylene compounds of formula (I):

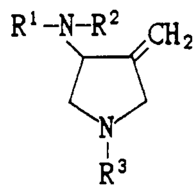

( I )

wherein $R^1$ and $R^3$ each is an amino-protecting group, and $R^2$ is hydrogen or a lower alkyl, characterized by subjecting a ketone of formula (II):

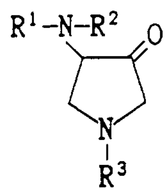

( II )

wherein $R^1$, $R^2$, and $R^3$ are the same as defined above, to methylenation.

This invention is explained in more detail below.

The methylenation of the ketones (II) may be performed by, for example, (1) a Wittig-type reaction described in, e.g., Witting et al., Chem. Ber., 87, 1318 (1954) or Org. React., 25, 73 (1977), in which such an organic phosphorous ylide as $(C_6H_5)_3P=CH_2$ or $(RO)_2P(O)=CH_2$ (In the formula, R is phenyl or lower alkyl) is used as a methylenation reagent (the reaction can be usually conducted at a temperature of -20 ° C to 100 ° C for several minutes to several hours using as a reaction solvent, for example, hydrocarbons such as toluene, ethers such as tetrahydrofuran and dimethoxyethane, or aprotic polar solvents such as dimethylsulfoxide and HMPA);

(2) a Grignard-type reaction or Peterson-type reaction described in, e.g ., F. Bertini et al., Tet., 26, 1281 (1970) or D. Peterson et al., J.A.C.S., 97, 1464 (1975), in which a nucleophilic reagent such as $CH_2(MgX')_2$ - (In the formula, X' is halogen), or $(CH_3)_3SiCH_2Li$ or $(CH_3)_3SiCH_2MgX'$ (In the formula, X' is as defined above) is used for methylenation (the reactions can be usually conducted at a temperature of -50 ° C to 100 ° C for several minutes to several hours using as a reaction solvent, for example, ethers such as tetrahydrofuran, diethyl ether, and dimethoxyethane, or hydrocarbons such as benzene and toluene); or

(3) reactions described in, e.g., H. Nozaki et al., Bull. Chem. Soc. Jap., 53, 1698 (1980), L. Lombardo, Tet. Lett., 23, 4293 (1982), or J. M. Tour et al., Tet. Lett., 30, 3927 (1989), in which metallic zinc such as $CH_2X'_2$-Zn-Lewis acid (wherein X' is as described above), e.g., $CH_2Br_2$-Zn-$TiCl_4$, and $CH_2X'_2$-Zn-organometallic compound (wherein X' is as described above), e.g., $CH_2Br_2$-Zn-$Cp_2ZrCl_2$, is used as a methylenation reagent (the reactions can be usually conducted at a temperature of -50 ° C to 100 ° C for several minutes to several hours using as a reaction solvent, for example, ethers such as tetrahydrofuran, diethyl ether, and dimethoxyethane, or hydrocarbons such as benzene and toluene).

The starting material of formula (II) can he prepared from 3-pyrroline. More particularly, it can be prepared by subjecting 3-pyrroline to N-protection, epoxidation with a peracid, chain-opening reaction by use of an amine ($R^2NH_2$), N-protection, and subsequent Swan oxidation or an alternative oxidation with a chromate (such as PCC, Jones reagent and the like) as follows.

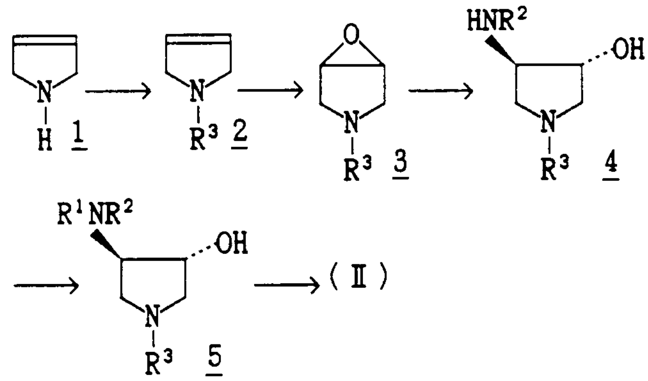


In the reaction scheme, $R^1$, $R^2$, and $R^3$ are the same as defined before.

All the amino-protecting groups may be used for those as represented by $R^1$ and $R^3$, if they can be deprotected without any catalytic reduction and are described, for example, in "Protective Group in Organic Chemistry" edited by J. F. W. McOmie, 1973, pp. 44-74. Preferred examples of the amino-protecting groups include acyl derivatives such as benzoyl, acetyl, formyl, and trifluoroacetyl; urethane-type derivatives such as benzyloxycarbonyl, t-butoxycarbonyl, isopropoxycarbonyl, and methoxycarbonyl; and alkyl derivatives such as allyl, benzyl, trityl, and tetrahydropyranyl.

As the lower alkyl represented by $R^2$, there can be mentioned $C_1$-$C_5$ alkyl such as methyl, ethyl, propyl, isopropyl, butyl, and pentyl.

This invention will be further explained by the following examples and reference example, which are not intended to restrict the scope of this invention.

Example 1

Preparation of 1-t-butoxycarbonyl-3-(N-methoxycarbonyl-N-methyl)-amino-4-metylenepyrrolidine Ia

35.0 g (0.13 mol) of 1-t-butoxycarbonyl-3-(N-methoxycarbonyl-N-methyl)amino-4-oxopyrrolidine IIa, 68.0 g (0.13 x 8 mol) of zinc dust, 45.0 g (0.13 x 1.2 mol) of zirconocene dichloride ($Cp_2ZrCl_2$), and 20 ml (0.13 x 2.2 mol) of dibromomethane are added to 250 ml of tetrahydrofuran, and the mixture is stirred for 1 hour under ice-cooling. This reaction mixture is added with 500 ml of toluene, and then filtered. The filtrate is concentrated to a volume of 500 ml, and washed with a saturated aqueous solution of sodium carbonate. The organic layer is subjected to silica gel chromatography (silica gel: 300 g; eluent: toluene, then toluene/ethyl acetate = 5/1) to give 17.0 g of the title compound Ia as an oil. The yield is 49%.

Example 2

To a suspension (10 ml) of 1.52 g (5 x 5 mmol) of zinc dust in tetrahydrofuran is added 770 $\mu$l (5 x 2.2 mmol) of dibromomethane, and the mixture is stirred at -60°C. After adding 7.5 ml (5 x 1.5 mmol) of a dichloromethane solution of 1.0 M $TiCl_4$ thereto the temperature of the mixture is elevated up to 0°C over 10 minutes. Then, 5 ml of a tetrahydrofuran solution containing 1.36 g (5.0 mmol) of the ketone compound IIa is added thereto, and stirred for 3 hours at room temperature. To this reaction mixture, ether and water are added, and the ether layer is washed with water. After drying, the solvent is removed by evaporation under reduced pressure. The residue is subjected to chromatography with Lover® column B (eluent: toluene/ethyl acetate = 5/1), thereby obtaining 500 mg of the oily title compound Ia as an oil. The yield is 33%.

Physical constants of compound Ia

| Elementary Analysis (for $C_{11}H_{20}N_2O_2$) | | | |
|---|---|---|---|
| Calcd.(%): | C, 62.24; | H, 9.50; | N, 13.20; |
| Found (%): | C, 61.96; | H, 9.43; | N, 13.20. |

IR (film)νmax 1700 $cm^{-1}$.
200 MHz-NMR($CDCl_3$) δ ppm 1.47 (s, 9H), 2.76 (s, 3H), 3.27 (br.t, J = 8Hz, 1H), 3.75 (m, 1H), 3.74 (s, 3H), 4.02 (br.s, 2H), 4.98 (m, 1H), 5.19 (br.s, 1H), 5.37 (br.s, 1H).

Reference Example 1

Preparation of 1-t-butoxycarbonyl-3-(N-methoxycarbonyl-N-methyl)-amino-4-oxopyrrolidine IIa

(1) To 100 ml of a solution containing 34.0 g of 3-pyrroline in methanol, t-butyloxycarboxylic anhydride is added under ice-cooling, and the mixture is stirred for 5.0 hours at room temperature, followed by removal of the solvent under reduced pressure. The residue is dissolved in 500 ml of dichloromethane, and 120 g of m-chloroperbenzoic acid (85%) are portionwise added thereto over 2 days at room temperature. The precipitate is filtered off, and the filtrate is concentrated, after which a toluene/hexane (1:1) mixture is added thereto, and precipitates are removed by filtration. The filtrate is washed with a diluted aqueous solution of sodium thiosulfate, a diluted aqueous solution of sodium bicarbonate, and then water. The solvent is removed by evaporation to leave 98 g of 1-t-butoxycarbonyl-3,4-epoxypyrrolidine 3. The yield is quantitative.

(2) First, 2.50 g of the epoxy compound 3 is dissolved in 20 ml of a 40% aqueous solution of methylamine, and the solution is allowed to stand for 2 days at room temperature. The reaction mixture is evaporated to dryness under reduced pressure, and the residue is crystallized from ethyl ether, thereby obtaining 2.30 g of 1-t-butoxycarbonyl-4-hydroxy-3-methylaminopyrrolidine 4. Mp. 98 - 99 °C. The yield is 79%.
IR (Nujol)νmax 3300, 3070, 1685 $cm^{-1}$.
200 MHz-NMR ($CDCl_3$) δ ppm 1.46 (s, 9H), 2.46 (s, 3H), 3.00-3.30 (m, 3H), 3.60-3.75 (m, 2H), 4.10 (dt, J = 5.5, 1H).

(3) To 40 ml of a solution containing 2.61 g of the amino alcohol 4 in tetrahydrofuran, 1.80 ml of triethylamine and 852 µl of chloromethyl carbonate are added at -30 °C, and the mixture is stirred for 20 minutes at room temperature. The solvent is removed by evaporation under reduced pressure, and ether is added to the residue. After washing with water, the solvent is removed by evaporation, thereby obtaining 2.80 g of viscous 1-t-butoxycarbonyl-4-hydroxy-3-(N-methoxycarbonyl-N-methyl)aminopyrrolidine 5. The yield is quantitative.
IR (film)νmax 3410, 1680-1710 $cm^{-1}$.
200 MHz-NMR ($CDCl_3$) δ ppm 1.46 (s, 9H), 2.86 (s, 3H), 3.20-3.40 (m, 3H), 3.73 (s, 3H), 4.20 (m, 1H), 4.25-4.50 (m, 2H).

(4) First, 5.0 ml of a solution containing 0.4 ml (4.4 mmol) of oxalyl dichloride in dichloromethane is cooled to -50 °C, and 0.7 ml (9.6 mmol) of DMSO is added to this solution, and the mixture is stirred for 5 minutes at -30 °C. To this mixture, 4 ml of a solution containing 1.10 g (4.0 mmol) of the N-carbamate compound 5 in dichloromethane is added, followed by stirring for 15 minutes at -20 °C. Further, 3 ml of triethylamine is added thereto, and the temperature of the mixture is allowed to increase up to room temperature over 10 minutes. After washing with water, the mixture is dried, and the solvent is removed by evaporation under reduced pressure, thereby obtaining 1.05 g of the viscous title compound IIa. The yield is 96%.
JR (film)νmax 1765, 1690 $cm^{-1}$.
200 MHz-NMR ($CDCl_3$) δ ppm 1.48 (s, 9H), 2.97 (s, 3H), 3.30-3.55 (m, 1H), 3.71 (s, 3H), 3.80-3.95 (m, 2H), 4.00-4.40 (m, 2H).

When the amino compound Ia obtained in the aforementioned Examples is subjected to deprotective reaction, and then allowed to react and treated as in Examples described in Japanese Laid-open Patent Publication No. 2-191273, the amino compound Ia provides a substituent moiety at the 7-position of pyridone carboxylic acid derivatives which have excellent antibacterial activity.

**Claims**

**1.** A process for the production of exo-methylene compounds of the formula (I):

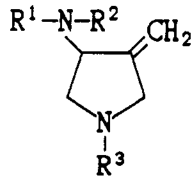

(I)

wherein $R^1$ and $R^3$ each is an amino-protecting group, and $R^2$ is hydrogen or a lower alkyl, which is characterized by methylenation of ketones of the formula:

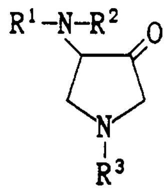

wherein $R^1$, $R^2$, and $R^3$ are the same as defined above.